# DEMANDE DE BREVET EUROPEEN

(11) **EP 3 772 528 A1**
(43) Date de publication de la demande: **10.02.2021**
(21) Numéro de dépôt: 19315089.3
(22) Date de dépôt: 08.08.2019
(51) Int. Cl.: C09B 55/00, C09B 69/00, C09K 11/06, G01N 33/533

(54) **UTILISATION DE COMPOSÉS FLUOROPHORES DE TYPE AZA-BODIPY COMME AGENTS DE CONTRASTE DANS L'INFRAROUGE TRÈS LOINTAIN**

(71) Demandeur: Université Grenoble Alpes, 38400 Saint Martin d'Hères (FR); Université de Bourgogne, 21000 Dijon (FR); Centre National de la Recherche Scientifique, 75016 Paris (FR); INSERM (Institut National de la Santé et de la Recherche Médicale), 75013 Paris (FR)
(72) Inventeur: Sancey, Lucie, 38850 Villages DU LAC DE PALADRU (FR); Goze, Christine, 21560 Remilly-sur-Tille (FR); Bodio, Ewen, 21850 Saint-Apollinaire (FR); Busser, Benoît, 38700 LE Sappey-EN-Chartreuse (FR); Pliquett, Jacques, 12621 Berlin (DE); Godard, Amélie, 21000 Dijon (FR); Kalot, Ghadir, 38400 Saint-Martin-D'Hères (FR); Josserand, Véronique, 38700 LA Tronche (FR); Le Guével, Xavier, 38000 Grenoble (FR); Coll, Jean-Luc, 38700 Le Sappey-en-Chartreuse (FR); Denat, Franck, 21800 Quetigny (FR)
(74) Mandataire: Novagraaf Technologies

(57) **Abrégé**

La présente invention se rapporte à l'utilisation comme agent de contraste, dans la fenêtre optique allant de 1000 à 1700 nm, d'un composé fluorophore aza-BODIPY. L'invention se rapporte aussi à l'utilisation comme agent de contraste d'une composition comprenant ledit composé fluorophore et un excipient pharmaceutiquement acceptable et/ou un solvant, à un kit comprenant un système d'injection et ledit fluorophore ou ladite composition et aussi à une méthode d'identification d'une cible biologique (telle qu'une cellule saine ou tumorale, une protéine, de l'ADN, ARN par exemple).

## Description

### Domaine technique

La présente invention appartient au domaine technique de l'imagerie optique et au domaine des agents de contraste, et plus particulièrement au domaine des agents de contraste organiques capables d'émettre dans la gamme de longueur d'onde la gamme de détection 1000-1700 nm, correspondant à l'infrarouge très lointain.

La présente invention se rapporte à l'utilisation comme agent de contraste, dans la fenêtre optique allant de 1000 à 1700 nm, d'un composé fluorophore aza-BODIPY. L'invention se rapporte aussi à l'utilisation comme agent de contraste d'une composition comprenant ledit composé fluorophore et un excipient pharmaceutiquement acceptable et/ou un solvant, à un kit comprenant un système d'injection et ledit fluorophore ou ladite composition et aussi à une méthode d'identification *in vitro* ou *in vivo* d'une cible biologique (telle qu'une cellule saine ou tumorale, une protéine, de l'ADN, ARN, un lipide, ou toute autre partie cible biologique animale ou végétale).

Dans la description, les références entre crochets ([]) renvoient à la liste des références présentée à la fin des exemples.

### État de la technique

L'imagerie optique connaît un nouvel essor avec l'arrivée de caméra à haute sensibilité dans la gamme de détection de 900 à 1700 nm, gamme qui correspond à l'infrarouge très lointain appelé NIR II ou SWIR (de l'anglais Short Wave Infrared). Cette gamme de détection est particulièrement intéressante en imagerie optique puisqu'elle permet théoriquement d'observer des signaux de fluorescence situés plus profondément dans le tissu (par rapport à l'imagerie NIR I), et/ou de manière plus résolue et plus sensible. Ces observations décrites dans les articles Bruns et *al*, Carr et al et Thimsen et *al* **[1],** sont notamment possibles du fait de la moindre auto-fluorescence des tissus dans cette gamme optique.

En dessous de 1000 nm, de nombreuses molécules organiques sont décrites comme agent de contraste, notamment la gamme des Cyanines, Alexa, Atto, BODIPY, DyLight, Rhodamine, Fluoresceine, Indocyanine green, etc. De plus, il existe des composés inorganiques tels que les QDOT utilisables en imagerie optique dans ces plages optiques. Les composés « visibles » dans les gammes optiques allant de 1000 à 1700 nm ont l'avantage d'émettre dans une gamme optique qui améliore la résolution et la profondeur de détection par rapport aux gammes optiques inférieures à 1000 nm.

Avec l'arrivée de ces nouvelles caméras et divers outils optiques adaptés aux longueurs d'ondes comprises entre 1000 et 1700 nm (comme les lentilles et objectifs), le développement d'agents de contraste adaptés devient nécessaire.

Il existe donc un réel besoin d'identifier des agents de contrastes pouvant être utilisés avec ces nouveaux outils et permettre ainsi de grandement améliorer la qualité des images observées, d'identifier plus facilement des cellules ciblées (par exemple tumorales), et d'élargir la gamme de détection dans les analyses de multi-fluorescence.

Il est du mérite de la demanderesse d'avoir identifié une classe de composés fluorophores, nommés aza-BODIPY, capable d'émettre dans la gamme de détection 1000 à 1700 nm, seuls, encapsulés ou greffés à une molécule d'intérêt (par exemple anticorps ou ligand biologique), ou une cellule d'intérêt (par exemple macrophage ou cellule souche).

L'utilisation des agents de contraste selon l'invention permet en outre de suivre la distribution de cibles biologiques en tube, *in vitro* ou *in vivo.*

D'autres avantages pourront encore apparaître à l'homme du métier à la lecture des exemples ci-dessous, illustrés par les figures annexées, donnés à titre illustratif et non limitatif.

### Exposé de l'invention

L'invention se rapporte à l'utilisation comme agent de contraste dans la fenêtre optique allant de 1000 à 1700 nm d'un composé fluorophore de formule I : 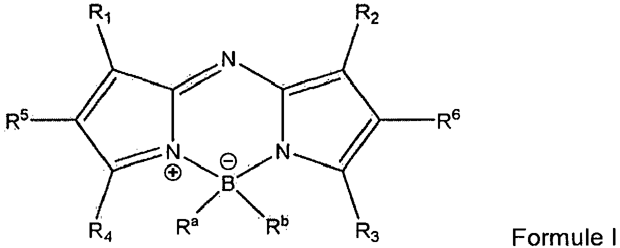 dans laquelle,
- R¹, R², R³ et R⁴, identiques ou différents, représentent un groupe aryle ou hétéroaryle en C5 à C7, éventuellement substitué par au moins un groupe choisi parmi un halogène, -NR^{c}R^{d}, -OR^{d}, hydrazine, -CF₃ et-CN,
- au moins un parmi R¹, R², R³ et R⁴ est un groupe aryle en C5 à C7 substitué par un groupe -NR^{c}R^{d}, et éventuellement un groupe choisi parmi un halogène, -OR^{d}, hydrazine, -CF₃ et-CN,
- R⁵ et R⁶, identiques ou différents représentent un hydrogène, un halogène, un groupe en C1 à C15 comprenant une fonction aldéhyde, cétone, acide carboxylique ou ester, un nitrile, -SO₃Na, un groupe vinyle éventuellement substitué par un groupement cétone, ester ou aromatique, une imine substituée par un groupement alkyle ou aromatique, un groupe alcyne éventuellement substitué par un groupement alkyle ou un aromatique, SPh, un chalcogène aromatique (SePh, TePh), un amide, un groupe aryle ou hétéroaryle en C5 à C7, éventuellement substitué par au moins un groupe choisi parmi un halogène, -NR^{c}R^{d}, -OR^{d}, hydrazine, -CF₃ et -CN,
- optionnellement R³ et R⁵ et/ou R⁴ et R⁶ sont liés de manière covalente et forment ensemble un groupe aryle ou hétéroaryle en C5 à C7, éventuellement substitué par au moins un groupe choisi parmi un halogène, -NR^{c}R^{d}, -OR^{d}, hydrazine, -CF₃ et -CN,
- R^{c} et R^{d}, identiques ou différents, représentent un hydrogène ou une chaine alkyle, linéaire ou ramifiée en C1 à C3,
- R^{a} et R^{b}, identiques ou différents, représentent :
   - un halogène, de préférence choisi dans le groupe comprenant le fluor et le chlore,
   - un groupe en C1 à C50, aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O ,N, P et/ou S, de préférence sous la forme d'une ou plusieurs fonctions hydrophiles choisies parmi les fonctions ammonium quaternaire, sulfate, sulfonate et phosphonate,
   - un groupe en C1 à C50, aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O, N, P et/ou S, de préférence sous la forme d'une ou plusieurs fonctions bioconjugables choisies parmi amine, acide carboxylique, ester activé de type N-hydrosuccinimide, pentafluorophényle, tetrafluorophenyle, squarate et plus particulièrement diéthylsquarate, maléimide, thiol, isothiocyanate, isocyanate, oxadiazolyl méthyl sulfone, azoture, tetrazine substituée ou non substituée, triazole, trans-cyclooctène, cyclooctyne et plus particulièrement dibenzocyclooctyne, bicyclononyne et un complexe PPh₂AuCl,
   - un vecteur biologique couplé de manière covalente via un groupe en C1 à C50, aliphatique ou hétéroaliphatique, linéaire ou ramifié, saturé ou insaturé, comprenant éventuellement un ou plusieurs hétéroatomes choisis parmi O, N, P et/ou S, de préférence sous la forme d'une ou plusieurs fonctions bioconjugables telles que définies ci-dessus,
   - un complexe métallique à visée thérapeutique, formé d'un agent chélatant et d'un métal,
   - un complexe radiométallique, formé d'un agent chélatant et d'un radiométal, ou
   - une molécule dont le diamètre hydrodynamique est inférieur à 10 nm, un peptide cyclique ou linéaire, un anticorps, un fragment d'anticorps, un nanobody, un affibody, un aptamère, une courte séquence d'ADN ou d'ARN, un sucre, un polysaccharide, un acide aminé, une vitamine, une molécule de type AMD3100, un ligand de la PSMA, un stéroïde (par exemple la progestérone), un acide gras (par exemple en C4 à C36), une polyamine (par exemple en C4 à C14), un polyphénol, une base de l'ADN ou un dérivé de la caféine.

Avantageusement, le composé fluorophore peut être sous forme de sel ou de sel pharmaceutiquement acceptable.

Dans le cadre de la présente invention, on entend par « fenêtre optique », un intervalle de longueur d'onde. Les fluorophores utilisés comme agent de contraste selon l'invention peuvent émettre dans une fenêtre optique allant de 1000 à 1700 nm, de préférence de 1000 à 1300 nm, et de manière encore plus préférée de 1000 à 1100 nm.

On entend par « cible biologique », une cellule, saine ou pathologique, une organelle, un constituant de cellule animale ou végétale de type protéine, lipide, ADN/ARN, mais aussi un anticorps, un constituant de la matrice extracellulaire ou un constituant d'un liquide biologique.

On entend par « agent de contraste », une molécule ou substance qui augmente artificiellement le contraste permettant de visualiser une structure anatomique (par exemple, un organe) ou pathologique (par exemple, une tumeur) naturellement peu ou pas contrastée, et que l'on aurait donc du mal à distinguer dans son environnement. Dans le cadre de la présente invention, les agents de contrastes émettent dans l'intervalle allant de 1000 nm à 1700 nm, appartenant à l'infrarouge très lointain, également appelé NIR II ou SWIR.

On entend par « BODIPY », des composés comprenant le motif de bore-dipyrométhène, principalement connus comme étant des colorants absorbant fortement dans l'UV et ayant la propriété d'émettre une fluorescence étroite avec un rendement quantique important. Ils dérivent tous du 4,4-difluoro-4-bora-3a, 4a-diaza-s-indacène :

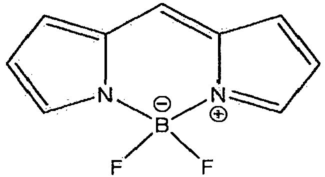

On entend par « aza-BODIPY », des composés BODIPY comprenant un atome d'azote en position 8 :

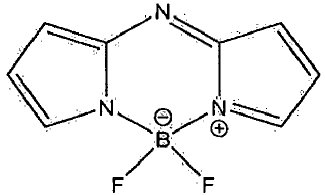

Dans le cadre de la présente invention, on entend par « aliphatique » désigne les groupes non aromatiques. Les groupes aliphatiques peuvent être cycliques. Les groupes aliphatiques peuvent être saturés, comme l'hexane, ou insaturés, comme l'hexène et l'hexyne. Les groupes à chaîne ouverte (qu'ils soient droits ou ramifiés) ne contiennent aucun type de cycle et sont donc aliphatiques. Les groupes aliphatiques peuvent être saturés, reliés par des liaisons simples (alcanes) ou insaturés, avec des liaisons doubles (alcènes) ou triples (alcynes). Les groupes « hétéroaliphatiques » sont des groupes aliphatiques portant un ou plusieurs hétéroatomes, les plus courants étant l'oxygène, l'azote, le phosphore et le soufre.

Le terme « dérivé » désigne un composé chimique ou une molécule fabriquée à partir d'un composé parent par une ou plusieurs réactions chimiques.

En général, le terme « substitué » ou « non substitué », précédé ou non du terme « éventuellement » ou « optionnellement », et les substituants contenus dans la formule de cette invention, désignent le remplacement des radicaux hydrogène dans une structure donnée par le radical d'un substituant spécifié. Lorsque plus d'un poste dans une structure donnée peut être remplacé par plus d'un substituant choisi dans un groupe donné, le substituant peut être le même ou différent à chaque poste. Tel qu'utilisé ici, le terme « substitué » est envisagé pour inclure tous les substituants autorisés des composés organiques.

Tel qu'utilisé ici, le terme « alkyle » se réfère aux groupes alkyles linéaires et ramifiés. Une convention analogue s'applique à d'autres termes génériques tels que « alcényle », « alcynyle », etc. Les groupes alkyles illustratifs comprennent, entre autres, les groupes méthyle, éthyle, n-propyle, isopropyle, allyle, n-butyle, sec-butyle, isobutyle, tert-butyle, n-pentyle, sec-pentyle, isopentyle, tert-pentyle, n-hexyle, sec-hexyle, et autres, qui peuvent aussi porter un ou plusieurs substituants. Les groupes alcényle comprennent, sans toutefois s'y limiter, l'éthényle, le propényle, le butényle, le 1-méthyl-2-butényl-2-I-yle, etc. Les groupes alcynyles représentatifs comprennent, sans s'y limiter, l'éthynyle, le 2-propynyle (propargy1), le 1-propynyle et autres.

Tel qu'utilisé ici, les termes « comprenant éventuellement un ou plusieurs hétéroatomes », se réfèrent aux groupes portant ou ayant inclus dans la chaîne principale des hétéroatomes choisis parmi O, N, P et S.

En général, les termes « saturé » ou « insaturé », tels qu'ils sont utilisés ici, désignent les groupes dont la structure moléculaire contient une ou plusieurs doubles liaisons carbone-carbone ou triple liaisons.

En général, les termes « groupe aromatique », « cycle ou hétérocycle aromatique » ou « aryle » ou « hétéroaryle », tels qu'ils sont utilisés ici, désignent des groupes hydrocarbonés mono- ou polycycliques insaturés, stables, substitués ou non, ayant de préférence 3-14 atomes de carbone, comprenant au moins un cycle satisfaisant la règle de Hückel pour l'aromaticité. Exemples de groupe aromatiques : phényle, indanyle, indényle, indényle, naphtyle, phénanthryle et anthracyle, sans toutefois s'y limiter.

En général, « cyclique » ou « non cyclique », tel qu'utilisé ici, se réfère à un fragment cyclique à 3 à 8 chaînons, dans la chaîne principale ou sur une chaîne latérale, substitué ou non substitué et comprenant éventuellement un ou plusieurs hétéroatomes. Les exemples d'un tel groupement hétéroaryle comprennent, sans s'y limiter, les suivants : pyridinyle, thiazolyle, thiazolyle, thiényle, furanyle, pyrrolyle, pyrazolyle, imidazolyle, triazolyle, tétrazolyle, benzofuranyle, benzazépinyle, thianaphtalényle, indolyle, indolinyle, quinolinyle, isoquinolinyle, benzimidazolyle, tétrahydroquinoléinyle, tétrahydroquinoléinyle, tétrahydroisoquinoléinyle, triazinyle, thianthrène, isobenzofuranyle, chroményle, xanthényle, phénoxanthinyle, isothiazolyle, isoxazolyle, pyrazinyle, pyridazinyle, indolizinyle, isoindolyle, indazolyle, purinyle, quinoléinyle, phtalazinyle, naphtyridinyle, quinoxalinyle, quinazolinyle, cinnolinyle, ptéridinyle, carbazolyle, β-carbolinyle, phénanthridinyle, acridinyle, pyrimidinyle, phénanthrolinyle, phénazinyle, phénothiazinyle, furazanyle, phenoxazinyle, isochromanyle, chromanyle, imidazolidinyle, imidazolinyle, pyrazolidinyle, pyrazolinyle, pyrazolinyle, indolinyle, isoindolinyle, oxazolidinyle, benzotriazolyle, benzisoxazolyle, oxindolyle, benzoxazolinyle, benzothiényle, benzothiazolyle, isatinyle, dihydropyridyle, pyrimidinyle, s-triazolinyle, oxazolyle et thiofuranyle.

Généralement, le terme « indépendamment » désigne le fait que les substituants, atomes ou groupes auxquels ces termes font référence sont choisis dans la liste des variables indépendamment les uns des autres (c'est-à-dire qu'ils peuvent être identiques ou différents).

Le terme « halogène » utilisé ici désigne un atome choisi parmi le fluor, le chlore, le brome et l'iode, de préférence le fluor et le chlore.

On entend par une « fonction bioconjugable », une fonction chimique permettant de lier de manière covalente les composés de l'invention à une molécule d'intérêt, et plus particulièrement une molécule d'intérêt biologique, de préférence un vecteur biologique. Les exemples d'une telle fonction, sans s'y limiter, sont les suivants : amine, acide carboxylique, ester activé de type N-hydrosuccinimide, pentafluorophényle, tetrafluorophenyle, fonction de type squarate et plus particulièrement diéthylsquarate, maléimide, thiol, isothiocyanate, isocyanate, oxadiazolyl méthyl sulfone, azoture, tetrazine substituée ou non, triazole, trans-cyclooctène, fonction de type cyclooctyne et plus particulièrement dibenzocyclooctyne, bicyclononyne et un complexe PPh₂AuCl.

On entend par « vecteur biologique », tout système d'encapsulation, ou tout ligand permettant de reconnaître une cible biologique spécifique couplé de manière covalente ou non via une fonction bioconjugable.

On entend par « agent chélatant », toute substance chimique qui a la propriété de fixer durablement des ions pour former un complexe, et plus particulièrement les cations métalliques. Il peut s'agir notamment des agents chélatants de la famille des polyamines, cycliques ou non.

On entend par « radiométal », un métal radioactif émettant par exemple des rayonnements gamma ou beta+, tel que le Gallium-68 ou le Fluor-18 par exemple, ou encore des rayonnements beta- ou alpha, comme par exemple le Lutécium-177 ou l'Actinium-225.

On entend par « complexe métallique à visée thérapeutique », tout complexe incluant un atome possédant des propriétés thérapeutiques, à lui seul ou après activation par un stimulus externe ou interne. Il peut s'agir par exemple d'un complexe choisi parmi PR₂M (M = Ru(II), Os (II), Ru(III), Au(I) ou (III),Pt(II), Pt(IV), Pd(II), Ir(III), Cu(I), Cu(II)) (R= alkyle, aryle, hétéroaryle de préférence triazaphosphaadamantane), carbène-M (M = Ru(II), Os (II), Ru(III), Au(I) ou (III),Pt(II), Pt(IV), Ir(III), Cu(I), Cu(II)), phénylpyridine-M (M = Au(III), Pt(II), Ru(II), Ir(III), Re(V), Re(III), Cu(II)), polypyridine (M = Au(III), Pt(II), Ru(II), Ir(III), Re(V), Re(III), Cu(II), Os(II)) S-M (M =Au(I), Au(III), Cu(I), Cu(II), Ti(IV), Zr(IV), alcyne Au(I), dithiocarbamate-M (M = Au(I), Au(III), Cu(I), Cu(II), quinoléine-M (M = Ga, Fe), η3-arène-M (M = Ru(II),Os(II),Cr(VI),Mo(III)), métallocène-M (M = Fe(II), Fe(III), Ti(IV), Ti(III), Zr(IV), Ir(III), Rh(III), Cr(VI), Ta(III), Os(II)), salen et salan-M (M = Au(III), Ti(IV), Zr(IV), Cu(II), Pt(II), Pd(II), dérivés de malonate-M (M = Pt(II),Ti(IV)), éthylène diamine -M (M = Pt(II), Pd(II), Cu(II), Au(III), Ru(II), Os(II)), benzaldimine -M (M = Ru(II), Rh(III), Ir(III)).

De la même façon, on entend par « complexe radiométallique », tout complexe contenant un atome métallique radioactif. Le complexe peut être composé d'un agent chélatant choisi parmi les dérivés de DTPA, NOTA, NODAGA, DOTA, DOTAGA, p-NCS-Bn-DOTA, p-NCS-Bn-NOTA, DFO, sarcophagine, cyclame ponté, salan, salen, HBED, polypyridines de type bipyridine, terpyridine, phenanthroline, phosphine ou diphosphine, carbene, arene, cyclopentadiene, alcyne, thiolate, phénylpyridine, phényltriazole et d'un radiométal choisi parmi Ga68, Ga67, AIF18, In111, Zr89, Sc43, Sc44, Sm153, Cu61, Cu64, Co55, Co57, Tb152, Tb157, Ru103, Ru97, Ru95, Os191 Au198, Au199, Ti45, Pt195, Pt193, Pd100, Re186, Re188. Il peut s'agir par exemple de type DOTA ou NODAGA et d'un radiométal choisi parmi Ga68 et In111 pour obtenir des sondes bimodales détectables en imagerie PET/SPECT et optique ou bien encore choisi parmi Lu177, Y90, Ac225, Pb212, Bi212, Eb109, Yt161, Sc47, Cu67, Tb161, Os191, Pt195, Pt193, Au199, Pd103, Re186, Re188, Sm153 pour des applications théranostiques ; tel que le DOTAGA-¹¹¹In par exemple.

Comme l'entend l'homme du métier, tous les nombres, y compris ceux qui expriment les quantités d'ingrédients, les propriétés telles que le poids moléculaire, les conditions de réaction, etc. sont des approximations et sont compris comme étant éventuellement modifiés dans tous les cas par le terme « environ ». Ces valeurs peuvent varier en fonction des propriétés recherchées par les personnes compétentes qui utilisent les enseignements des descriptions qui suivent. Il est également entendu que ces valeurs contiennent une variabilité inhérente résultant nécessairement des écarts-types trouvés dans leurs mesures d'essai respectives.

L'homme du métier reconnaîtra aussi facilement que lorsque les membres sont regroupés de la même manière, par exemple dans un groupe Markush, l'invention englobe non seulement l'ensemble du groupe énuméré, mais chaque membre du groupe individuellement et tous les sous-groupes possibles du groupe principal. En outre, à toutes fins utiles, l'invention englobe non seulement le groupe principal, mais aussi le groupe principal en l'absence d'un ou de plusieurs des membres du groupe. L'invention prévoit donc l'exclusion explicite d'un ou de plusieurs membres d'un groupe récité. En conséquence, des réserves peuvent s'appliquer à l'une quelconque des catégories ou incarnations divulguées, en vertu desquelles un ou plusieurs des éléments, espèces ou incarnations récités peuvent être exclus de ces catégories ou incarnations, par exemple, lorsqu'ils sont utilisés dans une limitation négative explicite.

Tel qu'utilisé ici, le terme « isomère » désigne les composés qui peuvent exister sous une ou plusieurs formes géométriques, optiques, énantiomères, diastéréoisomères, épimères, atropes, stéréoisomères, tautomères, conformationnelles ou anomères particulières. Exemples d'isomères limités aux formes cis et trans ; formes E et Z ; formes c, t et r ; formes c, t et r ; formes endo- et exo- ; formes R, S et méso- ; formes D et L ; formes d et I ; formes d et I ; (+) et (-) ; formes céto-, énol et énolate ; syn- et antiformes ; formes synclinales et anticlinales ; formes α- et β- ; formes axiales et équatoriales ; formes bateau-, chaise, torsion, enveloppe et demi-chaise ; et leurs combinaisons, ci-après dénommées collectivement « isomères » (ou « formes isomériques »). Dans la présente divulgation, le terme « isomère » exclut les isomères structuraux ou les isomères constitutionnels dont la constitution diffère et qui sont décrits par une formule linéaire différente **[2].**

Avantageusement, le composé fluorophore peut être choisi parmi les composés de formule I dans laquelle R¹, R², R³ et R⁴ sont identiques ou différents. R¹, R², R³ et R⁴ peuvent indépendamment représenter un groupe aryle ou hétéroaryle en C5 à C7. R¹, R², R³ et R⁴ peuvent indépendamment éventuellement être substitués par au moins un groupe choisi parmi un halogène, -NR^{c}R^{d}, -OR^{d}, hydrazine, -CF₃ et-CN.

Le composé fluorophore peut être choisi parmi les composés de formule I dans laquelle au moins un parmi R¹, R², R³ et R⁴ est un groupe aryle en C5 à C7 substitué par un groupe -NR^{c}R^{d} et éventuellement un groupe choisi parmi un halogène, -OR^{d}, hydrazine, -CF₃ et -CN. De préférence, lorsqu'il s'agit d'un groupe aryle en C6, le groupe -NR^{c}R^{d} est en position para.

Avantageusement, le composé fluorophore est choisi parmi les composés de formule I dans laquelle R⁵ et R⁶ sont identiques ou différents. R⁵ et R⁶ peuvent indépendamment représenter un hydrogène, un halogène, un groupe en C1 à C15 comprenant une fonction aldéhyde, cétone, acide carboxylique ou ester, un nitrile, -SO₃Na, un groupe vinyle éventuellement substitué par un un groupement cétone, ester ou aromatique, une imine substituée par un groupement alkyle ou aromatique, un groupe alcyne éventuellement substitué par un groupement alkyle ou un aromatique, SPh, un chalcogène aromatique (SePh, TePh), un amide, un groupe aryle ou hétéroaryle en C5 à C7, éventuellement substitué par au moins un groupe choisi parmi un halogène, -NR^{c}R^{d}, -OR^{d}, hydrazine,-CF₃ et-CN. De préférence, R⁵ et R⁶ sont un hydrogène ou -SO₃Na.

Avantageusement, le composé fluorophore est choisi parmi les composés de formule I dans laquelle R^{c} et R^{d} sont identiques ou différents. R^{c} et R^{d} peuvent indépendamment représenter un hydrogène ou une chaine alkyle, linéaire ou ramifiée en C1 à C3. De préférence, -NR^{c}R^{d} est choisi dans le groupe comprenant -NH₂, -NMe₂, -NEt₂, -NPr₂, de préférence -NMe₂.

Avantageusement, R³ et R⁵ et/ou R⁴ et R⁶ peuvent être liés de manière covalente et former ensemble un groupe aryle ou hétéroaryle en C5 à C7, éventuellement substitué par au moins un groupe choisi parmi un halogène, -NR^{c}R^{d}, -OR^{d}, hydrazine, -CF₃ et -CN.

Avantageusement, le composé fluorophore peut être choisi parmi les composés de formule I dans laquelle R⁵ et R⁶ sont un hydrogène.

Avantageusement, le composé fluorophore peut être choisi parmi les composés de formule I dans laquelle R¹ et R², identiques ou différents, représentent un groupe aryle en C5 à C7 substitué par un groupe -NR^{c}R^{d} et éventuellement un groupe choisi parmi un halogène, -OR^{d}, hydrazine,-CF₃ et -CN. De préférence, le composé fluorophore peut être choisi parmi les composés de formule I dans laquelle R¹ et/ou R² représentent un groupe phényle substitué par un groupe -NR^{c}R^{d}, de préférence en position para.

Avantageusement, le composé fluorophore peut être choisi parmi les composés de formule I dans laquelle R^{a} et R^{b} sont identiques ou différents. R^{a} et R^{b} peuvent représenter un halogène, de préférence le fluor ou le chlore.

Avantageusement, le composé fluorophore peut être choisi parmi les composés de formule I dans laquelle R^{a} et R^{b} sont identiques ou différents. R^{a} et R^{b} peuvent représenter un groupe en C1 à C50, de préférence en C2 à C30 aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O, N, P et/ou S, de préférence sous la forme d'une ou plusieurs fonctions hydrophiles choisies parmi ammonium quaternaire, sulfate, sulfonate et phosphonate.

Avantageusement, le composé fluorophore peut être choisi parmi les composés de formule I dans laquelle R^{a} et R^{b} sont identiques ou différents. R^{a} et R^{b} peuvent représenter un groupe en C1 à C50, de préférence en C5 à C30, aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O, N, P et/ou S, de préférence sous la forme d'une ou plusieurs fonctions bioconjugables choisies parmi amine, acide carboxylique, ester activé de type N-hydrosuccinimide, pentafluorophényle, tetrafluorophenyle, squarate et plus particulièrement diéthylsquarate, maléimide, thiol, isothiocyanate, isocyanate, oxadiazolyl méthyl sulfone, azoture, tetrazine substituée ou non substituée, triazole, trans-cyclooctène, cyclooctyne et plus particulièrement dibenzocyclooctyne et bicyclononyne et un complexe PPh₂AuCl.

Avantageusement, le composé fluorophore peut être choisi parmi les composés de formule I dans laquelle R^{a} et R^{b} sont identiques ou différents. R^{a} et R^{b} peuvent représenter un vecteur biologique couplé de manière covalente via un groupe comprenant une fonction bioconjugable tel que défini ci-dessus.

Avantageusement, le composé fluorophore est choisi parmi les composés de formule I dans laquelle R^{a} et ou R^{b}, identiques ou différents, comprennent une ¹⁰BSH (Na₂B₁₂H₁₁SH ou borocaptate sodium, enrichi en bore10), pour l'obtention de théranostiques actifs en borothérapie.

Avantageusement, le composé fluorophore peut être choisi parmi les composés de formule I dans laquelle R^{a} et R^{b} sont identiques ou différents. R^{a} et R^{b} peuvent représenter un complexe métallique à visée thérapeutique, de préférence choisi parmi les complexe de formule PR₂M dans laquelle M est un métal choisi dans le groupe comprenant Ru(II), Os (II), Ru(III), Au(I) ou (III),Pt(II), Pt(IV), Pd(II), Ir(III), Cu(I), Cu(II) et R est un groupe alkyle, aryle, hétéroaryle en C1 à C12, de préférence triazaphosphaadamantane, carbène-M dans laquelle M est un métal choisi dans le groupe comprenant M = Ru(II), Os (II), Ru(III), Au(I) ou (III),Pt(II), Pt(IV), Ir(III), Cu(I), Cu(II), phénylpyridine-M dans laquelle M est un métal choisi dans le groupe comprenant Au(III), Pt(II), Ru(II), Ir(III), Re(V), Re(III), Cu(II), polypyridine-M dans laquelle M est un métal choisi dans le groupe comprenant Au(III), Pt(II), Ru(II), Ir(III), Re(V), Re(III), Cu(II), Os(II), S-M dans laquelle M est un métal choisi dans le groupe comprenant Au(I), Au(III), Cu(I), Cu(II), Ti(IV), Zr(IV), alcyne-Au(I), dithiocarbamate-M dans laquelle M est un métal choisi dans le groupe comprenant Au(I), Au(III), Cu(I), Cu(II), quinoléine-M dans laquelle M est un métal choisi dans le groupe comprenant Ga, Fe, η3-arène-M dans laquelle M est un métal choisi dans le groupe comprenant Ru(II), Os(II), Cr(VI), Mo(III)), métallocène-M dans laquelle M est un métal choisi dans le groupe comprenant Fe(II), Fe(III), Ti(IV), Ti(III), Zr(IV), Ir(III), Rh(III), Cr(VI), Ta(III), Os(II)), salen et salan-M dans laquelle M est un métal choisi dans le groupe comprenant Au(III), Ti(IV), Zr(IV), Cu(II), Pt(II), Pd(II), dérivés de malonate-M dans laquelle M est un métal choisi dans le groupe comprenant Pt(II), Ti(IV), éthylène diamine-M dans laquelle M est un métal choisi dans le groupe comprenant Pt(II), Pd(II), Cu(II), Au(III), Ru(II), Os(II), benzaldimine-M dans laquelle M est un métal choisi dans le groupe comprenant Ru(II), Rh(III), Ir(III).

Avantageusement, le composé fluorophore peut être choisi parmi les composés de formule I dans laquelle R^{a} et R^{b} sont identiques ou différents. R^{a} et R^{b} peuvent représenter un complexe radiométallique formé d'un agent chélatant et d'un radiométal. Le complexe peut être composé d'un agent chélatant choisi parmi les dérivés de DTPA, NOTA, NODAGA, DOTA, DOTAGA, p-NCS-Bn-DOTA, p-NCS-Bn-NOTA, DFO, sarcophagine, cyclame ponté, salan, salen, HBED, polypyridines de type bipyridine, terpyridine, phenanthroline, phosphine ou diphosphine, carbene, arene, cyclopentadiene, alcyne, thiolate, phénylpyridine, phényltriazole et d'un radiométal choisi parmi Ga68, Ga67, AIF18, In111, Zr89, Sc43, Sc44, Sm153, Cu61, Cu64, Co55, Co57, Tb152, Tb157, Ru103, Ru97, Ru95, Os191 Au198, Au199, Ti45, Pt195, Pt193, Pd100, Re186, Re188. Il peut s'agir par exemple de type DOTA ou NODAGA et d'un radiométal choisi parmi Ga68 et In111 pour obtenir des sondes bimodales détectables en imagerie PET/SPECT et optique ou bien encore choisi parmi Lu177, Y90, Ac225, Pb212, Bi212, Eb109, Yt161, Sc47, Cu67, Tb161, Os191, Pt195, Pt193, Au199, Pd103, Re186, Re188, Sm153, pour des applications théranostiques.

Avantageusement, le composé fluorophore peut être choisi parmi les composés de formule I dans laquelle R^{a} et R^{b}, identiques ou différents, sont choisis parmi une petite molécule, c'est-à-dire une molécule dont le diamètre hydrodynamique est inférieur à 10 nm tels que des clusters d'or ou des complexes métalliques, un peptide cyclique ou linéaire (tel que le c(RGDfK) ciblant l'intégrine αᵥβ₃ ou l'ATWLPPR ciblant la neuropiline), un anticorps (par exemple de type anti-CD44), un fragment d'anticorps ou un nanobody ciblant un récepteur membranaire ou intracellulaire, un affibody, un aptamère, une courte séquence d'ADN ou d'ARN, un sucre (comme par exemple un thioglucose ou thioglucose peracétylé) ou un polysaccharide, un acide aminé, une vitamine (par exemple de type acide folique), un ligand de type AMD3100, un ligand de la PSMA, un stéroïde, un acide gras, une polyamine (par exemple de type spermine, spermidine, cadavérine ou putrécine), un polyphénol (par exemple resvératrol), une base de l'ADN, un dérivé de la caféine, la progestérone.

Avantageusement, le composé fluorophore peut être choisi parmi les composés de formule I dans laquelle R^{a} et R^{b}, identiques ou différents, sont choisis parmi un halogène, de préférence le fluor.

Avantageusement, le composé fluorophore peut être choisi parmi les composés de formule I dans laquelle R^{a} et R^{b}, identiques ou différents, sont choisis parmi les groupes hydrophiles de formules suivantes :

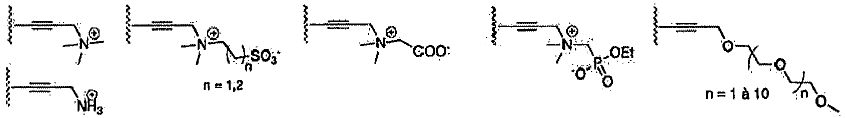

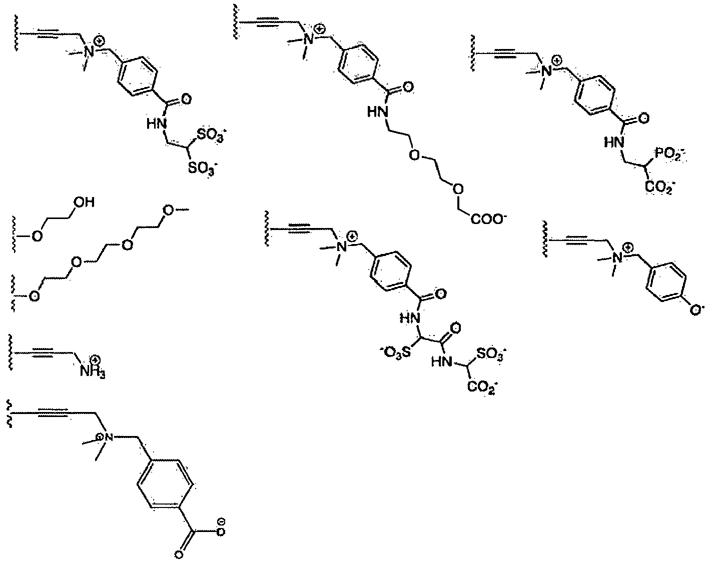

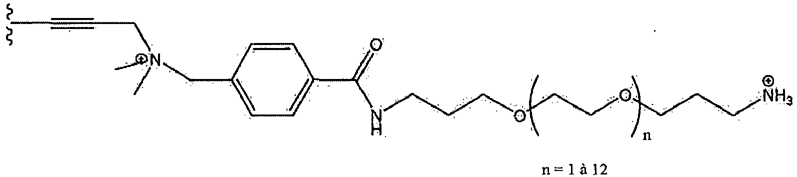

Dans la présente demande, le symbole 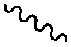 , représente le point de rattachement du groupe représenté à la molécule. Par exemple, il peut s'agir du point de rattachement au B (atome de bore).

Avantageusement, le composé fluorophore peut être choisi parmi les composés de formule I dans laquelle R^{a} et R^{b}, identiques ou différents, sont choisis parmi les groupes comprenant une fonction bioconjugable de formules suivantes : 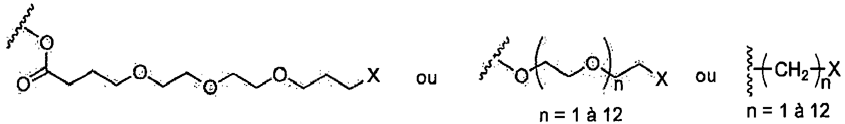 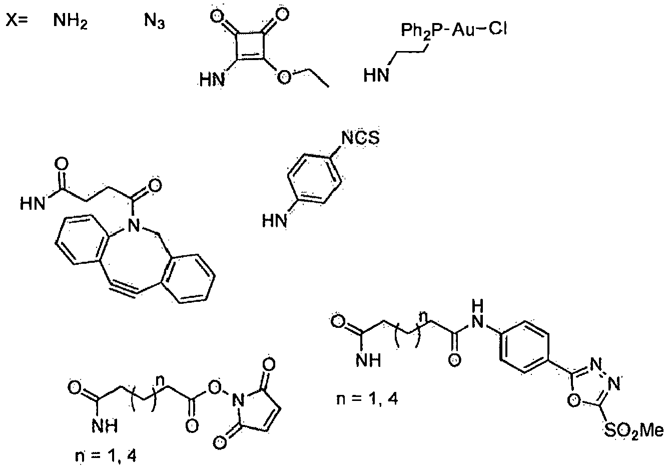 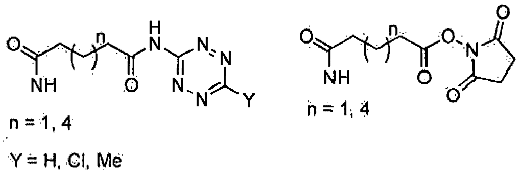 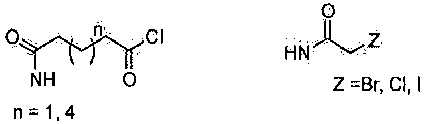 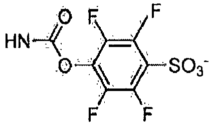 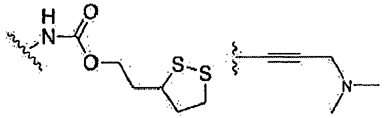 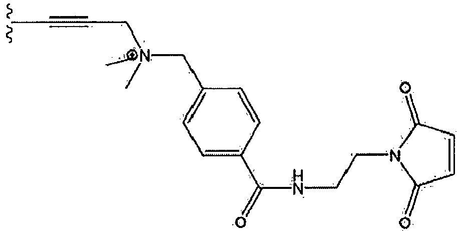 -NH₂ et -Si(OMe)₃.

De préférence, la fonction bioconjugable peut être choisie parmi les fonctions N-hydroxysuccinimide, isothiocyanate conjuguée, tétrazine, diéthylsquarate, maléimide, oxadiazolyl méthyl sulfone, pentafluorophenyl, azoture, un complexe PPh₂AuCl et NH₂.

Avantageusement, le composé fluorophore peut être choisi parmi les composés de formule I, dans laquelle R^{a} et/ou R^{b} comprennent un vecteur biologique couplé de manière covalente et répondent, par exemple de formule suivante :

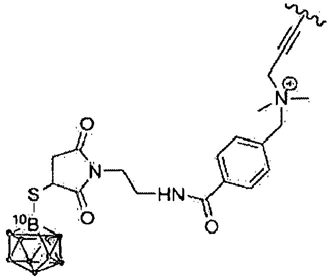

Avantageusement, le composé fluorophore peut être choisi parmi les composés de formule I dans laquelle R^{a} et R^{b}, identiques ou différents, sont choisis parmi PPh₂-Au(I), PPh₂-Pt(II), PPh₂-Pt(IV), phneylpyridine-Au(III).

Avantageusement, le composé fluorophore peut être choisi parmi les composés de formule I dans laquelle R^{a} et R^{b}, identiques ou différents, sont choisis parmi DOTA-In(III), DOTAGA-In(III), NODAGA-Cu(II), NODAGA-Ga(III).

Avantageusement, le composé fluorophore peut être choisi parmi les composés de formule I dans laquelle R^{a} et R^{b}, identiques ou différents, sont choisis parmi un peptide cyclique ou linéaire (tel que le c(RGDfK) ciblant l'intégrine αᵥβ₃ ou l'ATWLPPR ciblant la neuropiline), un anticorps (par exemple de type anti-CD44), un fragment d'anticorps ou un nanobody ciblant un récepteur membranaire ou intracellulaire, une courte séquence d'ADN ou d'ARN, un sucre (comme par exemple un thioglucose ou thioglucose peracétylé) ou un polysaccharide, un acide aminé, une vitamine (par exemple de type acide folique), un ligand de type AMD3100, un ligand de la PSMA, un stéroïde (par exemple la progestérone), un acide gras (par exemple en C4 à C36), une polyamine (par exemple de type spermine, spermidine, cadavérine ou putrécine), un polyphénol (par exemple resvératrol), une base de l'ADN, un dérivé de la caféine (par exemple la caféine).

Avantageusement, le composé fluorophore peut être choisi parmi les composés fluorophores de formule I dans laquelle R⁵ et R⁶ sont H, R¹ et R² sont 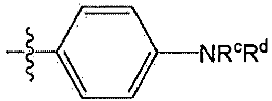 et R³ et R⁴ sont respectivement 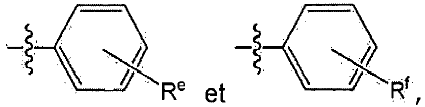 de formule II : 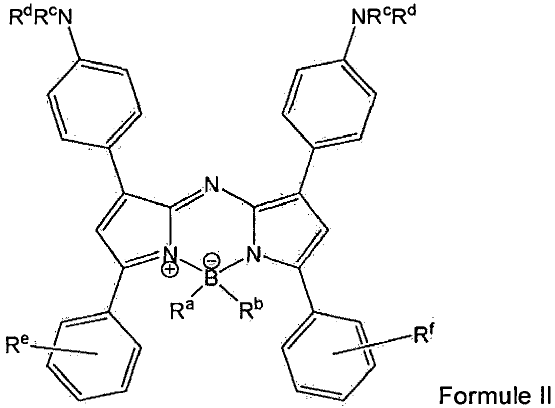 dans laquelle,
- R^{a}, R^{b}, R^{c}, R^{d} ont les définitions données ci-dessus,
- R^{e} et R^{f}, identiques ou différents, représentent au moins un groupe parmi un hydrogène, un halogène, -NR^{c}R^{d}, -OR^{d}, hydrazine, -CF₃ et -CN.

Avantageusement, le composé fluorophore peut être choisi parmi les composés de formule I ou II dans laquelle R^{c} et R^{d} sont -CH₃.

Avantageusement, le composé fluorophore peut être choisi parmi les composés de formule II dans laquelle R^{e} et R^{f} sont H ou -OMe, de préférence en position para.

Avantageusement, le composé fluorophore peut être compris dans une composition comprenant en outre un excipient pharmaceutiquement acceptable et/ou un solvant. Il peut s'agir de tout excipient pharmaceutiquement acceptable que l'homme du métier pourra mettre dans la composition pour modifier, son pH, son osmolarité, sa viscosité ou sa solubilité. Il peut s'agir par exemple de NaCl (aqueux à 0,9%), de solutions glucosées à 5g/L, d'eau ppi ou bien encore de solutions tamponnées telles que le PBS ou d'autres tampons pharmaceutiquement acceptables.

Avantageusement, la composition peut avoir un pH compris dans un intervalle allant de 4 à 10, de préférence de 6 à 8 ou bien encore de 6,8 à 7,6. De préférence le pH de la composition est de 7,4.

Avantageusement, la concentration en composé fluorophore dans la composition est comprise dans un intervalle allant de 0,1 à 1000 µmol/L, de préférence de 1 à 100 µmol/L ou encore de 10 à 40 µmol/L (ces valeurs sont notamment compatibles avec des détections *in vitro*) ou dans un intervalle allant de 4 nmol/kg à 300 µmol/kg chez la souris, de préférence de 40 nmol/kg à 12,5 µmol/kg, ou encore autour de 1 à 1,5 µmol/kg (ces valeurs sont notamment compatibles avec des détections *in vivo*).

Avantageusement, le composé fluorophore peut être encapsulé. Dans le cadre de l'invention, on entend par « encapsulé » tout objet biologiquement compatible permettant de regrouper et éventuellement de protéger plusieurs composés fluorophores et de les conduire vers la cible d'intérêt, tels que des nanoparticules lipidiques, des nanoformulations notamment à base de polysaccharides, des nanotubes de carbone, des micelles. Par exemple, le composé fluorophore peut être encapsulé dans une formulation lipophile comprenant un liposome tel que décrit dans la publication Gravier et al **[3],** une capsule lipidique telle que décrite dans la publication Hirsjärvi et al **[4]** ou des nanodomaines hydrophobes tels que les nanoformulations de polysaccharides décrites dans la publication de Garcia et al **[5].**

Avantageusement, le composé fluorophore peut être lié de manière covalente à une nanoparticule. Le couplage peut être effectué via le groupe R^{a} ou R^{b} porteur d'une fonction bioconjugable telle que définie ci-dessus. Les nanoparticules pouvant être liées de manière covalente au composé de formule I ou II peuvent être du type petites nanoparticules de tailles inférieures à 10 kDa, ou les nanoparticules lipidiques. II peut s'agir par exemple de nanoclusters d'or ou de lipidots.

L'invention se rapporte également à un kit comprenant un système d'injection, et une composition comprenant un composé fluorophore de formule I ou II tel que défini ci-dessus et un excipient pharmaceutiquement acceptable et/ou un solvant.

L'invention se rapporte aussi à une méthode d'identification *in vitro* d'une cible biologique (telle qu'une cellule saine ou tumorale, une protéine, de l'ADN, ARN par exemple) comprenant au moins les étapes de :
- marquage des cellules d'un échantillon prélevé ou cultivé avec une composition comprenant un composé fluorophore de formule I ou II tel que défini ci-dessus,
- mesure de la fluorescence dans la fenêtre optique comprise dans un intervalle allant de 1000 à 1700 nm, et
- identification des cellules ciblées.

Avantageusement, dans la méthode d'identification *in vitro* selon l'invention, la concentration en fluorophore dans la composition est comprise dans un intervalle allant de 0,1 à 1000 µmol/L, de préférence de 1 à 100 µmol/L ou encore de 10 à 40 µmol/L.

Avantageusement, le marquage des cellules de l'échantillon est réalisé par injection ou par aspersion de la composition comprenant le fluorophore.

Avantageusement, la mesure de la fluorescence dans la fenêtre optique comprise dans un intervalle allant de 1000 à 1700 nm est réalisée par tout moyen connu de l'homme du métier et apte à mesurer ladite fluorescence. II peut s'agir par exemple de microscopie confocale ou d'épifluorescence cytométrie de flux, imagerie optique par réflexion de fluorescence (2D ou 3D) ou bien encore par une sonde optique pour l'aide à la chirurgie ou adaptée à la lecture de plaque multi-puits.

L'invention se rapporte aussi à une méthode d'identification *in vivo* d'une cible biologique (telle qu'une cellule saine ou tumorale, une protéine, de l'ADN, ARN par exemple) comprenant au moins les étapes de :
- marquage des cellules d'un sujet par injection ou aspersion avec une composition comprenant un composé fluorophore de formule I ou II tel que défini ci-dessus,
- mesure de la fluorescence dans la fenêtre optique comprise dans un intervalle allant de 1000 à 1700 nm, et
- identification des cellules ciblées.

Avantageusement, dans la méthode d'identification *in vivo* selon l'invention, la concentration en fluorophore dans la composition est comprise dans un intervalle allant de 4 nmol/kg à 300 µmol/kg chez la souris, de préférence de 40 nmol/kg à 12,5 µmol/kg, ou encore autour de 1 à 1,5 µmol/kg.

Avantageusement, le marquage des cellules de l'échantillon est réalisé par injection ou par aspersion de la composition comprenant le fluorophore.

Avantageusement, la mesure de la fluorescence dans la fenêtre optique comprise dans un intervalle allant de 1000 à 1700 nm est réalisée par tout moyen connu de l'homme du métier et apte à mesurer ladite fluorescence. Il peut s'agir par exemple de microscopies d'épifluorescence ou confocale, cytométrie de flux, imagerie optique par réflexion de fluorescence (2D ou 3D) ou bien encore par une sonde optique portable telles que celles utilisée pour l'aide à la chirurgie, ou adaptée à un lecteur de plaques.

Le modèle *in vivo* utilisé dans la présente est basé sur la souris. Avantageusement, dans la méthode selon l'invention, la concentration en fluorophore dans la composition administrée *in vivo* est comprise dans un intervalle allant de 4 nmol/kg à 300 µmol/kg chez la souris, de préférence de 40 nmol/kg à 12,5 µmol/kg, ou encore autour de 1 à 1,5 µmol/kg. Toute autre administration pourra se faire selon les conversions inter-espèces, tel que décrit par exemple dans l'article de Reagan-Shaw et al **[6].**

De manière non limitative, on peut citer les avantages liés à l'invention :
- augmentation de la qualité des images observées, amélioration de la résolution des images (par comparaison à l'imagerie NIR),
- identification plus facile des cellules tumorales, notamment l'observation de tumeurs in vivo,
- détection en profondeur du tissu de la cible biologique,
- possibilité de suivre la distribution de cibles biologiques en tube, *in vitro* ou *in vivo,*
- augmentation de la plage de longueurs d'onde utilisable dans des analyses de multi-marquage,
- extension de la gamme de composés fluorescents dans les marquages multiples,
- permettre de l'imagerie dans la gamme optique supérieure à 1000 nm appelée SWIR ou infrarouge très lointain,
- permettre également de coupler cette technique d'imagerie à l'imagerie par photoacoustique,
- diminution du scattering et de l'autofluorescence des tissus.

De plus, selon l'invention, l'agent de contraste optique peut être utilisé seul, ou couplé à une molécule d'intérêt, ou encapsulé dans une nano-formulation.

### Brève description des figures

La figure 1 représente une souris portant une tumeur U87MG au niveau de la patte arrière droite, avant et 24h après injection de aza-BODIPY (AG22). Les plans supérieurs sont enregistrés en imagerie NIR : Fluo800 correspond à une excitation à 780nm et une collection entre 830-900 nm. Les plans inférieurs sont excités à 830 nm (SWIR800) et l'émission est collectée à l'aide d'un filtre passe long 1064 nm, soit entre 1064 et 1700 nm.
La figure 2 représente les spectres d'émission des composés AG22 et AG04.

### EXEMPLES

### Exemple 1 : Synthèse d'agents de contraste selon l'invention

### Matériel & Méthodes

Les réactions ont été effectuées dans des solvants Carlo Erba de qualité technique sous atmosphère normale, sauf indications contraires. Les expériences exigeant des conditions anhydres ont été réalisées sous argon. Les solvants secs ont été achetés auprès de Carlo Erba, non stabilisés et ont été séchés à l'aide d'un MB-SPS-800 (MBraun) ou de PureSolv-MD-5 (Inert®). Tous les réactifs commerciaux ont été achetés auprès de Sigma-Aldrich® ou ACROS Organics® et ont été utilisés tels que reçus sans aucune purification. Le TOTA-Boc (boc-1-amino-4,7,10-trioxa-13-tridécanamine) a été acheté à Iris Biotech GmbH® et ¹⁰B-BSH à Katchem®. Le suivi des réactions a été effectué par HPLC-MS et par chromatographie sur couche mince sur des plaques de gel de silice Merck® 60 F254 de 0.2 mm d'épaisseur, révélées par UV (254 nm). Les purifications sur colonne de chromatographie ont été effectuées sur gel de silice Sigma-Aldrich® technique, 40-63 µm, 230-400 mesh, 60 Å.

Les spectres de RMN (¹H, ¹³C) ont été enregistrés sur un appareil Bruker 500 Avance III ou Bruker 600 Avance III HD (équipé de sondes à large bande à double résonance). Les déplacements chimiques sont exprimés en ppm et sont donnés par rapport au TMS (¹H, ¹³C) avec pour référence le signal du solvant résiduel. Les spectres de masse haute résolution ont été enregistrés sur un spectromètre Thermo LTQ Orbitrap XL ESI-MS. La RMN et les analyses de masse ont été effectuées au sein de la Plateforme d'Analyse Chimique et de Synthèse Moléculaire de l'Université de Bourgogne (PACSMUB).

Les analyses HPLC-MS ont été réalisées sur un instrument Thermo-Dionex Ultimate 3000 (pompe + échantillonneur automatique à 20 °C + four à colonne à 25 °C) équipé d'un détecteur à barrette de diodes (Thermo-Dionex DAD 3000-RS) et d'un simple MSQ Plus spectromètre de masse quadripolaire équipé d'une colonne Phenomenex Kinetex® (2,6 µm, C18, 100 Å, colonne LC 50 x 2,1 mm). Le gradient utilisé pour la caractérisation est le suivant (Gradient A) :

| Temps (min) | % H₂O (+0,1% acide formique) | % ACN (+0,1% acide formique) | Débit (mL/min) |
|---|---|---|---|
| 0 | 95 | 5 | 0.5 |
| 5 | 0 | 100 | 0.5 |
| 6,5 | 0 | 100 | 0.5 |
| 6,6 | 95 | 5 | 0.5 |
| 8,5 | 95 | 5 | 0.5 |
| 8,51 | 95 | 5 | 0.05 |

Les purifications par HPLC semi-préparative ont été effectuées sur un appareil HPLC de Shimadzu équipé de 2 pompes LC-20AT, d'un détecteur UV/Vis SPD-20A, d'un collecteur de fractions FRC-10A, d'un échantillonneur SIL-10AP et d'une unité de contrôle CBM-20A. La colonne utilisée est une colonne Shim-Pack GIST 5 µm C18 10x250 mm et les gradients utilisés sont les suivants :

| | **Gradient A** | | | **Gradient B** | | | |
|---|---|---|---|---|---|---|---|
| Temps (min) | %H₂O + 0,1%TFA | %ACN + 0,1%TFA | Debit (mL/min) | Temps (min) | % H₂O + 0,1 %TFA | % ACN + 0,1%TFA | Debit (mL/min) |
| 0 | 75 | 25 | 5 | 0 | 60 | 40 | 5 |
| 5 | 75 | 25 | 5 | 5 | 60 | 40 | 5 |
| 25 | 0 | 100 | 5 | 25 | 0 | 100 | 5 |
| 28 | 0 | 100 | 5 | 28 | 0 | 100 | 5 |
| 30 | 75 | 25 | 5 | 30 | 60 | 40 | 5 |

| **Gradient C** | | | | **Gradient D** | | | |
|---|---|---|---|---|---|---|---|
| Temps (min) | % H₂O + 0,1 %TFA | % ACN + 0,1 %TFA | Debit (mL/min) | Temps (min) | % H₂O + 0,1 %TFA | % ACN + 0,1 %TFA | Debit (mL/min) |
| 0 | 80 | 20 | 5 | 0 | 80 | 20 | 5 |
| 5 | 80 | 20 | 5 | 5 | 80 | 20 | 5 |
| 28 | 0 | 100 | 5 | 25 | 0 | 100 | 5 |
| 33 | 0 | 100 | 5 | 27 | 0 | 100 | 5 |
| 35 | 80 | 20 | 5 | 28 | 80 | 20 | 5 |

### Composé AG22

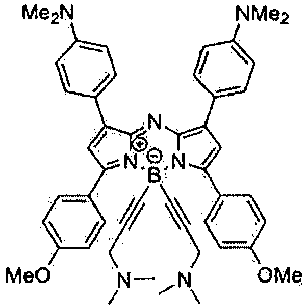

18 µL de *N,N*-diméthylpropargylamine (156 µmole, 2 éq) sont dissous dans 2 mL de THF (tétrahydrofurane) sous argon (verrerie shlenk). Le bromure d'éthyle magnésium (0,17 mL, 170 µmole, 2,2 éq) est ensuite additionné et le mélange est porté à reflux pendant 45 min, laissé revenir à température ambiante, puis transféré par canule dans un second schlenk contenant le précurseur aza-BODIPY (50 mg, 78 µmole, 1 éq). Le mélange est ensuite agité à reflux pendant 45 min sous argon, puis la réaction est stoppée par ajout de 2 mL d'EtOH. Les solvants sont éliminés par évaporation sous pression réduite. Le brut formé est solubilisé dans 10 mL d'AcOEt, puis 10 mL d'eau distillée sont ajoutés. Après agitation et décantation dans une ampoule à décanter, la phase organique est mise de côté et la phase aqueuse est extraite deux fois avec 10 mL d'AcOEt. Les phases organiques sont réunies, lavées deux fois avec 10 mL une solution aqueuse de NaHCO₃ diluée deux fois, puis séchées sur MgSO₄ anhydre. La solution résultante est filtrée et le solvant est éliminé par évaporation sous pression réduite. Le résidu est purifié par colonne de chromatographie sur gel de silice (éluant : 98/2 DCM/MeOH → 100% MeOH) pour isoler AG22 sous la forme d'une poudre violette pailletée (51,1 mg, 66,3 µmole, 85%).
**RMN ¹H** (CDCl₃, 500 MHz) δ (ppm) : 8,18 (d ; ³*J* = 8,9 Hz ; 4H) ; 8,05 (d ; ³*J* = 9,0 Hz ; 4H) ; 6,97 (d ; ³*J* = 9,0 Hz ; 4H) ; 6,77 (d ; ³*J* = 8,9 Hz ; 4H) ; 6,77 (s ; 2H) ; 3,86 (s ; 6H) ; 3,27 (s ; 4H) ; 3,08 (s ; 12H) ; 2,26 (s ; 12H).
**RMN ¹³C** {1H} (CDCl₃, 150 MHz) δ (ppm) : 160,8 ; 156,5 ; 150,8 ; 143,0 ; 142,4 ; 132,0 ; 130,6 ; 125,6 ; 121,0 ; 115,6 ; 113,4 ; 112,0 ; 55,4 ; 48,0 ; 42,3 ; 40,2.
**HR-MS** (ESI) (Da) : *m*/*z* calculée pour C₄₈H₅₂BN₇O₂ [M+H]⁺ 770,42755 ; trouvée 770,43559.
**HPLC analytique** (Gradient A) : Tr = 4.46 min.

### Composé AG24

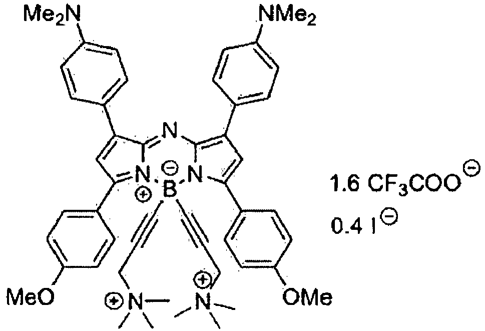

AG22 (30 mg, 39 µmole, 1 éq) est dissous dans 3 mL de DCM (dichlorométhane). L'iodométhane (1,5 mL, large excès) est ensuite ajouté et le milieu réactionnel est agité pendant 1h à température ambiante. Les solvants sont alors éliminés sous pression réduite et le brut obtenu est solubilisé dans 10 mL d'un mélange H₂O/DCM (1/1). Une extraction de la phase aqueuse par du DCM (3 × 5 mL) est réalisée, puis dans un deuxième temps la phase organique obtenue est extraite à l'eau (8 × 10 mL). La phase aqueuse récupérée est évaporée, et le précipité formé est purifié par HPLC (Gradient A). Le solide obtenu est lyophilisé pour fournir AG24 pur sous la forme d'un solide bleu-vert (17,8 mg, 17,2 µmole, 44%).
**RMN ¹H** (DMSO, 500 MHz) δ (ppm) : 8,24 (d ; ³*J*_{He-Hf} = 8,9 Hz ; 4H) ; 8,10 (d ; ³*J*_{Hb-Hc} = 9,0 Hz ; 4H) ; 7,22 (s ; 2H) ; 7,11 (d ; ³*J*_{Hb-Hc} = 9,0 Hz ; 4H) ; 6,86 (d ; ³*J*_{He-Hf} = 8,9 Hz ; 4H) ; 4,00 (s ; 4H) ; 3,87 (s ; 6H) ; 3,07 (s ; 12H) ; 2,78 (s ; 18H).
**HR-MS** (ESI) (Da) : *m*/*z* = calculée pour C₅₀H₅₈BN₇O₂²⁺ [M]²⁺ 399,73670 ; trouvée 399,73869.
**HPLC analytique** (Gradient A) : Tr = 4,49 min.

### Composé AG38

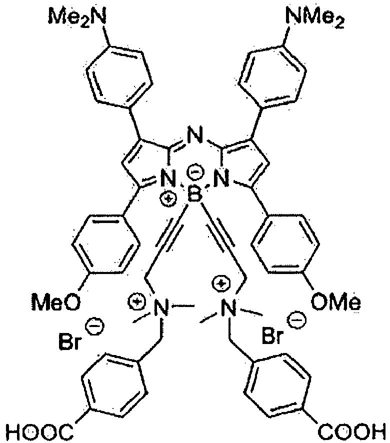

AG22 (250 mg, 0,32 mmole, 1 éq) est dissous dans 50 mL de THF et 8 mL d'H₂O. NaHCO₃ (137 mg, 1,63 mmole, 5,1 éq) est ajouté, suivi de l'acide 4-bromoéthylbenzoïque (144 mg, 0,67 mmole, 2,1 éq). Le mélange réactionnel est laissé sous agitation à température ambiante pendant une nuit. 90 mL d'Et₂O et 90 mL d'H₂O sont ensuite ajoutés et les phases organique et aqueuse sont séparées. La phase aqueuse est lavée à l'Et₂O (6 × 60 mL) de manière à éliminer les traces restantes d'acide 4-bromoéthylbenzoïque. La phase aqueuse est ensuite réduite à 1/3 de son volume initial par évaporation de l'eau à l'évaporateur rotatif (bain à 35°C). 10 mL d'acide chlorhydrique (3M) sont ensuite ajoutés. Le contenu du ballon est alors centrifugés. Le surnageant est retiré et le culot est mis en suspension dans 15 mL d'Et₂O et est de nouveau centrifugé. L'opération est renouvelée 3 fois et les culots obtenus sont ensuite solubilisés dans du MeOH et évaporés à sec à l'évaporateur rotatif (bain à 35 °C) afin d'obtenir AG38 pur sous la forme d'une poudre noire pailletée (336 mg, 0,28 mmole, 85%).
**RMN ¹H** (DMSO, 500 MHz) δ (ppm) : 8,30 (d ; ³*J* = 8,9 Hz ; 4H) ; 8,12 (d ; ³*J* = 9,0 Hz ; 4H) ; 7,95 (d ; ³*J* = 8,3 Hz ; 4H) ; 7,43 (d ; ³*J* = 8,3 Hz ; 4H) ; 7,28 (s ; 2H) ; 7,08 (d ; ³*J* = 9,0 Hz ; 4H) ; 6,88 (d ; ³*J* = 8,9 Hz ; 4H) ; 4,24 (s ; 4H) ; 3,96 (s ; 4H) ; 3,74 (s ; 6H) ; 3,08 (s ; 12H) ; 2,76 (s ; 12H).
**HPLC analytique** (Gradient A) : Tr = 4,50 min.

### Composé AG57

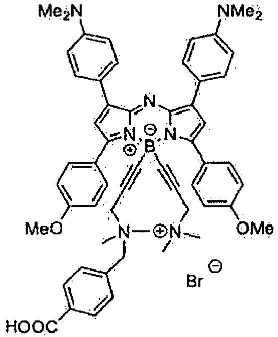

AG22 (75 mg ; 0,097 mmole, 1 éq) est dissous dans 60 mL de THF sec dans un ballon de 250 mL sous argon. L'acide 4-bromoéthylbenzoïque (23 mg ; 0,106 mmole, 1,1 éq) est ensuite ajouté et le mélange est laissé sous agitation à reflux pendant une nuit. Après refroidissement, le surnageant est retiré et le précipité formé est lavé au THF (3 × 15 mL), à l'éther diéthylique (2 × 15 mL) et au pentane (2 × 15 mL). L'ensemble des surnageants sont réunis et les solvants sont éliminés sous pression réduite. Le résidu obtenu est purifié par colonne chromatographique sur gel de silice (8:2 Toluène/MeOH → 100% MeOH) de manière à isoler AG57 sous la forme d'une poudre pailleté noire (20,7 mg, 0,021 mmole, 22%).
**RMN ¹H** (DMSO, 500 MHz) δ (ppm) : 8,36 (d ; ³*J* = 8,9 Hz ; 4H) ; 8,07 (d ; ³*J* = 9,0 Hz ; 4H) ; 7,98 (d ; ³*J* = 8,3 Hz ; 2H) ; 7,24 (d ; ³*J* = 8,3 Hz ; 2H) ; 7,00 (d ; ³ = 9,0 Hz ; 4H) ; 6,96 (s ; 2H) ; 6,82 (d ; ³*J* = 8,9 Hz ; 4H) ; 3,84 (s ; 2H) ; 3,75 (s ; 6H) ; 3,39 (s ; 2H) ; 3,25 (s ; 2H) ; 3,06 (s ; 12H) ; 2,58 (s ; 6) ; 2,24 (s ; 6H).
**HPLC analytique** (Gradient A) : 4,48 min.

### Composé AG46

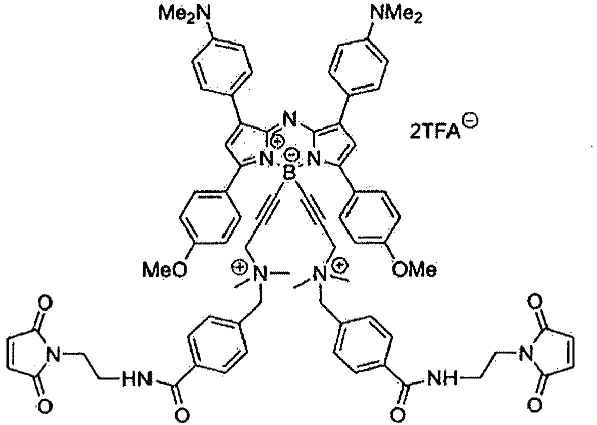

AG38 (250 mg, 0,209 mmole, 1 éq) est dissous dans 10 mL de DMF (diméthylformamide) anhydre dans un ballon de 100 mL. HBTU (208 mg, 0,548 mmole, 2,6 éq) est dissout dans 10 mL de DMF anhydre avant d'être ajouté au mélange réactionnel. 341 µL (1.959 mmole, 9,3 éq) de DIPEA (diisopropyléthylamine) sont ensuite additionnés et le mélange est laissé sous agitation à température ambiante pendant 1 h. 109.2 mg (0,618 mmole , 2,9 éq) de chlorhydrate de 2-aminoéthylmaléimide sont dissous dans 10 mL de DMF anhydre avant d'être ajoutés au milieu réactionnel qui est ensuite agité à température ambiante pendant une nuit, évaporé à sec puis purifié par HPLC semi-préparative (gradient 25% ACN → 100 % programme 30 min) pour isoler AG46 pur sous la forme d'un solide vert (151 mg, 0,133 mmole, 63%).
**RMN ¹H** (MeOD, 500 MHz) δ (ppm) : 8,36 (d ; ³*J* = 8,9 Hz ; 4H) ; 8,19 (d ; ³*J* = 8,9 Hz ; 4H) ; 7,72 (d ; ³ = 8,2 Hz ; 4H) ; 7,39 (d ; ³*J* = 8,2 Hz ; 4H) ; 7,22 (s ; 2H) ; 7,18 (d ; ³*J* = 8,9 Hz ; 4H) ; 7,09 (d ; ³*J* = 8,9 Hz ; 4H) ; 6,76 (s ; 4H) ; 4,18 (s ; 4H) ; 3,87 (s ; 4H) ; 3,76 (s ; 6H) ; 3,71 (dd ; ³*J* = 6,3 Hz ; ³*J* = 4,6 Hz ; 2H) ; 3,52 (dd ; ³*J* = 6,3 Hz ; ³*J* = 4,6 Hz ; 2H) ; 3,20 (s ; 12H) ; 2,84 (s ; 12H).
**RMN ¹³C (DMSO, 150 MHz) :** 171,1 ; 165,6 ; 160,9 ; 158,6 ; 158,3 ; 158,1 ; 157,8 ; 155,9 ; 151,0 ; 142,1 ; 141,6 ; 136,0 ; 134,6 ; 132,5 ; 132,0 ; 130,4 ; 130,1 ; 127,6 ; 124,3 ; 119,8 ; 116,7 ; 116,0 ; 114,7 ; 113,7 ; 112,1 ; 64,6 ; 55,4 ; 54,0 ; 49,1 ; 37,7 ; 37,1.
**HR-MS** (ESI) (Da) : *m*/*z* calculée pour C₇₆H₇₈BN₁₁O₈²⁺ [M]²⁺ 641,80585, trouvée 641,80752.
**HPLC-analytique** (Gradient A) : Tr = 4,56 min.

### Composé AG49

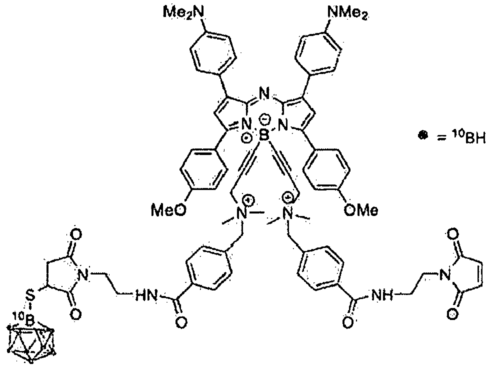

AG46 (120 mg ; 79 µmol ; 1 éq) est dissous dans 2 mL d'ACN (acétonitrile) dans un ballon de 10 mL. 42 mg (166 µmol ; 2,5 éq) de BSH sont ensuite ajoutés et la réaction est laissée sous agitation à 40°C pendant 48h. Le mélange réactionnel est transféré dans des tubes Falcons puis centrifugé. Le surnageant est retiré et le culot est de nouveau lavé avec de l'ACN (3 × 15 mL), avec du DCM (1 × 15 mL), de l'Et₂O (2 × 15 mL) et du pentane (2x 15 mL), ce qui permet d'isoler AG49 sous la forme d'un précipité bleu (62 mg, 41,9 µmol, 53%).
**RMN ¹H** (DMSO, 500 MHz) δ (ppm) : 8,65-8,63 (m ; 1H) ; 8 ;48-8,42 (m ; 1H) ; 8,33-8,30 (m ; 4H) ; 8,13-8,11 (m ; 4H) ; 7,82-7,75 (m ; 4H) ; 7,39-7,36 (m ; 4H) ; 7,28-7,27 (m ; 2H) ; 7,09-7,07 (m ; 4H) ; 7,00-6,99 (m ; 1H) ; 6,89-6,85 (m ; 4H) ; 4,17 (t ; ³*J* = 17,9 Hz ; 4H) ; 3,97-3,90 (m ; 4H) ; 3,74-3,73 (m ; 6H) ; 3,62 (dd ; ³*J* = 8,0 Hz ; ⁴*J* = 3,1 Hz ; 2H) ; 3,59-3,55 (m ; 2H) ; 3,54-3,48 (m ; 4H) ; 3,07 (s ; 12H) ; 3,03 (d ; ³*J* = 8,5 Hz ; 1H) ; 2,99 (d ; ³*J* = 8,5 Hz ; 1H) ; 2,78-2,73 (m ; 13H) ; 1,03 (bs ; 11H).
**HR-MS** (ESI) (Da) : *m*/*z* calculée pour C₇₆H₉₀B¹⁰B₁₂N₁₁Na²⁺O₈S²⁺ [M+2Na]²⁺ 746,90622, trouvée 746,90811.
**HPLC-analytique** (Gradient A) : Tr = 5,32 min.

### Composé AG58

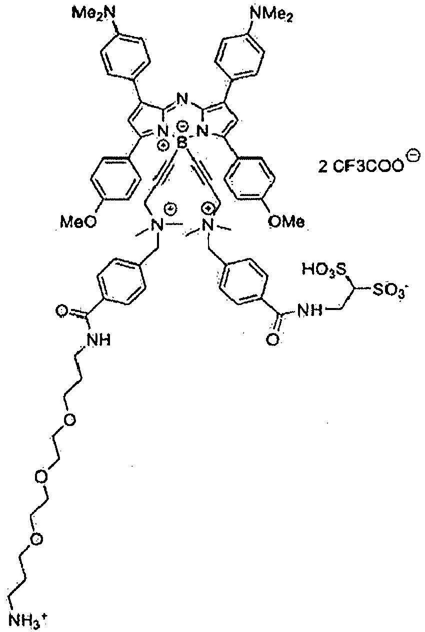

AG35-3 (35 mg ; 0,029 mmole ; 1 éq) est dissous dans 3 mL de DMF dans un ballon de 50 mL. 25 mg de HBTU (2-(1H-benzotriazol-1-yl)-1,1,3,3-tetramethyluronium hexafluorophosphate, Hexafluorophosphate Benzotriazole Tetramethyl Uronium) (0,067 mmole ; 2,3 éq) dissous dans 3 mL de DMF sont ensuite introduits, suivis de 40 µL de DIPEA (0,232 mmole , 8 éq). La réaction est agitée à température ambiante pendant 30 min sous argon. Une solution de H₂N-CH₂-CH-(SO₃⁻)₂ (sel de TBA ou tetrabutylammonium) à 0,5M (61 µL, 0,030 mmole, 1,1 éq) dans 3 mL de DMF est ajoutée au milieu réactionnel, qui est ensuite agité à température ambiante pendant 1 h. 14 mg (0,030 mmole, 1,1 éq) de TOTA-Boc dans 3 mL de DMF sont ensuite introduits dans la solution qui est encore agitée pendant 1h à température ambiante. Le mélange réactionnel est évaporé à sec, solubilisé dans 30 mL d'ACN, puis 15 mL d'HCl (3M) sont ajoutés. La réaction est agitée à 40°C pendant 2 h. Le brut réactionnel est évaporé à sec et purifié par HPLC semi-préparative (gradient 25% → 100%, programme 40 min), lyophilisé, permettant d'obtenir AG58 pur sous la forme d'une poudre verte (7 mg, 5 µmol, 18%).
**RMN ¹H** (DMSO, 500 MHz) δ (ppm) : 8,69 (t ; ³*J* = 4,4 Hz ; 1H) ; 8 ;50 (t ; ³*J* = 5,5 Hz ; 1H) ; 8,23 (d ; ³*J* = 8,9 Hz ; 4H) ; 8,12 (d ; ³*J* = 9,0 Hz ; 4H) ; 7,94 (d ; ³*J* = 8,3 Hz ; 2H) ; 7,90 (d ; ³*J* = 8,3 Hz ; 2H) ; 7,63 (d ; ³*J* = 8,3 Hz ; 2H) ; 7,60 (bs ; 3H) ; 7,56 (d ; ³*J* = 8,3 Hz ; 2H) ; 7,18 (s ; 2H) ; 6,98 (d ; ³*J* = 8,9 Hz ; 4H) ; 6,87 (d ; ³*J* = 8,9 Hz ; 4H) ; 4,60 (s ; 2H) ; 4,28 (s ; 2H) ; 3,95 (t ; ³*J* = 4,4 Hz ; 2H) ; 3,80 (s ; 2H) ; 3,72 (t ; ³*J* = 4,9 Hz ; 1H) ; 3,63 (s ; 6H) ; 3,51-3,41 (m ; 12H) ; 3,33 (dd ; ³*J* = 12,4 Hz ; ³*J* = 6,6 Hz ; 4H) ; 3,07 (s ; 12H) ; 3,06-3,01 (m ; 4H) ; 2,85-2,82 (m ; 2H) ; 2,72 (s ; 6H) ; 1,79-1,73 (m ; 4H) ; 1,60-1,55 (m ; 2H).
**HR-MS** (ESI) (Da) : *m*/*z* calculée pour C₇₆H₉₃BN₁₀O₁₃S₂²⁺ [M]²⁺ 714.32235, trouvée 714.32560.
**HPLC-analytique** (Gradient A) : Tr = 4,32 min.

### Composé AG60

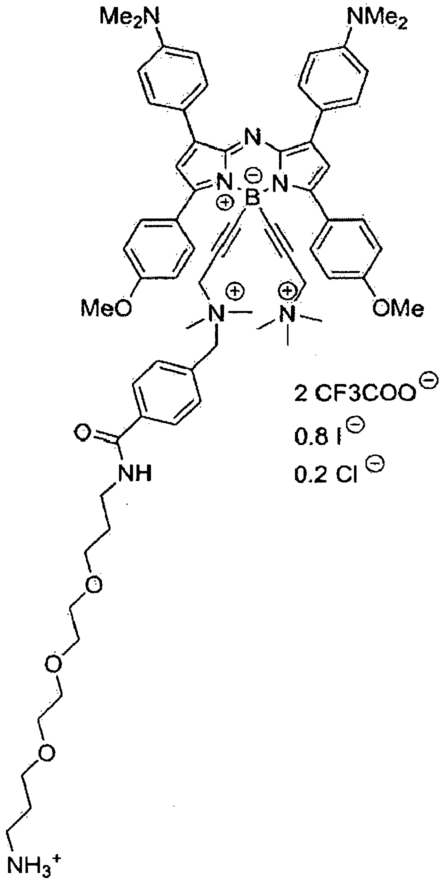

AG57-3 (21 mg ; 0,021 mmole ; 1 éq) est dissous dans 2 mL de DMF. 20 mg (0,052 mmole ; 2,5 éq) de HBTU sont dissous dans 2 mL avant d'être ajoutés à la solution d'AG57-3, suivis de 32 µL (0,160 mmole ; 8,6 éq) de DIPEA. Le mélange réactionnel est agité à 30°C sous argon. Au bout de 30 min, 10 mg (0,024 mmole ; 1,1 éq) de TOTA-Boc sont ajoutés puis la solution est agitée pendant 1h30 à 30°C. Les solvants sont éliminés sous pression réduite et le résidu résultant est purifié par colonne de chromatographie sur gel de silice (éluant : 100% DCM → 50/50 DCM/MeOH), permettant d'isoler l'intermédiaire TOTA sous la forme d'un solide noir.

Puis, dans un ballon de 25 mL sous argon, l'intermédiaire précédent est dissous dans 4 mL de DCM sec. Du iodométhane (1 mL, large excès) est additionné et la réaction est agitée à température ambiante pendant 1 h. Les solvants sont éliminés sous pression réduite, puis le résidu résultant est solubilisé dans 20 mL d'ACN. 10 mL d'une solution aqueuse d'HCl (3M) sont ajoutées et la réaction est laissée sous agitation à 40°C pendant 2 h. Le milieu réactionnel est évaporé à sec et est purifié par HPLC semi-préparative (gradient 20% ACN → 100% en 30 min), puis lyophilisé durant une nuit pour isoler AG60 pur sous la forme d'un solide bleu (4 mg, 2,4 µmol, 14%).
**RMN ¹H** (ACN, 500 MHz) δ (ppm) : 8,21-8,19 (m ; 2H) ; 8,18-8,15 (m ; 2H) ; 8,11-8,08 (m ; 4H) ; 7,89 (t ; ³*J* = 8,6 Hz ; 2H) ; 7,86 (s ; 1H) ; 7,52 (bs ; 3H) ; 7,36 (d ; ³*J* = 8,0 Hz ; 1 H) ; 7,32 (d ; ³*J* = 8,1 Hz ; 1 H) ; 7,04-7,01 (m ; 4H) ; 7,00 (d ; ³*J* = 8,7 Hz ; 2H) ; 6,89-6,86 (m ; 4H ) ; 4,01 (s ; 1H) ; 3,86 (s ; 1H) ; 3,85 (s ; 1H) ; 3,81 (s ; 1H) ; 3,80 (s ; 3H) ; 3,76 (s ; 3H) ; 3,66 (q ; *J* = 5,5 Hz ; 2H) ; 3,60-3,58 (m ; 4H) ; 3,57-3,55 (m ; 6H) ; 3,53 (t ; ³*J* = 5,8 Hz ; 2H) ; 3,44 (quin ; ³*J* = 6,4 Hz ; 3H) 3,35 (s ; 1H) ; 3,08 (s ; 12H) ; 2,83 (s ; 5H) ; 2,70 (s ; 3H) ; 2,66 (s ; 3H) ; 2,60 (s ; 3H) ; 1,90-1,85 (m ; 3H) ; 1,83-1,78 (m ; 2H).
**HR-MS** (ESI) (Da) : *m*/*z* calculée pour C₆₇H₈₅BN₉O₆³⁺ [M]³⁺ 374.22331, trouvée 374.22292.
**HPLC-analytique** (Gradient A) : Tr = 4,18 min.

### Exemple 2 : Mode opératoire d'imagerie de fluorescence sur petit animal

Des souris femelles NMRI Nu/Nu de 5 semaines sont placées en cage par 5, avec une alimentation et de l'eau at libitum, ainsi qu'un éclairage jour/nuit de 12h/12h, selon les recommandations éthiques en vigueur.

A l'âge de 6 semaines, des cellules tumorales U87MG (3 Millions / 100 µL) sont injectées aux souris en sous-cutané au niveau du membre inférieur droit : les animaux sont mis sous sédation par anesthésie gazeuse lors de cette injection. Les animaux sont placés de nouveau en cage pendant la durée du développement tumoral soit environ 3 semaines.

Lorsque la tumeur atteint environ 100 mm³ ou plus, la solution contenant le composé AG22 est injectée par voie intraveineuse (25 à 50 µg/souris), au niveau de la veine caudale de l'animal placé sous anesthésie gazeuse. Puis l'animal est réveillé pendant les phases externes à l'imagerie. Pour chaque session d'imagerie, l'animal est placé sous anesthésie gazeuse, l'imagerie est réalisée, puis l'animal retourne dans sa cage.

L'imagerie est réalisée grâce à une excitation à 830 nm puis le signal de fluorescence est collecté à l'aide d'un filtre passe long entre 1064 et 1700 nm.

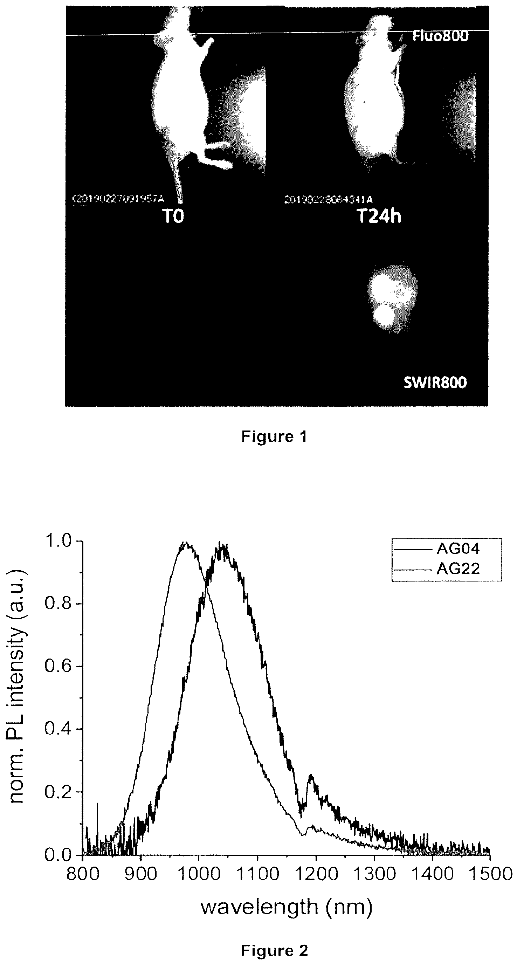

On observe (figure 1) la souris portant une tumeur U87MG au niveau de la patte arrière droite, avant et 24 heures après injection du composé AG22. Les plans supérieurs sont enregistrés en imagerie NIR. Fluo800 correspond à une excitation à 780 nm et une collection entre 830 et 900 nm. Les plans inférieurs sont excités à 830 nm (SWIR800) et l'émission est collectée à l'aide d'un filtre passe-long 1064 nm.

Les composés selon l'invention peuvent ainsi être observés dans les longueurs d'onde comprises dans un intervalle allant de 1000 à 1700 nm : ces longueurs d'onde facilitent la détection en profondeur, à meilleure résolution.

### Listes des références

[1] Bruns et al, Nat Biomed Eng. 2017; doi:10.1038/s41551-017-0056 / Carr et al, Proc Natl Acad Sci U S A. 2018 115(17):4465-70 / Thimsen et al, Nanophotonics 2017; 6(5): 1043-1054).
[2] PAC, 1996, 68, 2193 (Basic terminology of stereochemistry (IUPAC Recommendations 1996)) page 2205.
[3] Gravier et al, Mol Pharm. 2014 ; doi: 10.1021/mp500329z., page 3134.
[4] Hirsjärvi et al, Nanomedicine. 2013 ; doi: 10.1016/j.nano.2012.08. 005, page 376.
[5] Garcia et al, Biomater Sci. 2018 ; doi: 10.1039/c8bm00396c, page 1755.
[6] Reagan-Shaw et al, FASEB J, 2007, Vol 22 page 660, doi: 10.1096/fj.07-9574LSF

## Revendications

1. Utilisation comme agent de contraste dans la fenêtre optique de allant 1000 à 1700 nm d'un composé fluorophore de formule I : 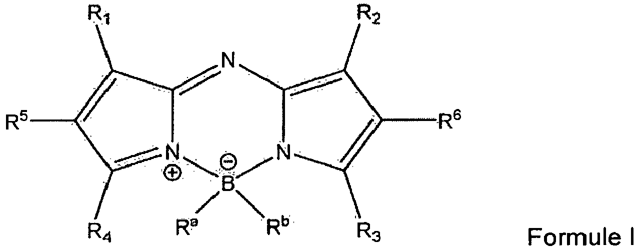
- R¹, R², R³ et R⁴, identiques ou différents, représentent un groupe aryle ou hétéroaryle en C5 à C7, éventuellement substitué par au moins un groupe choisi parmi un halogène, -NR^{c}R^{d}, -OR^{d}, hydrazine, -CF₃ et-CN,
- au moins un parmi R¹, R², R³ et R⁴ est un groupe aryle en C5 à C7 substitué par un groupe -NR^{c}R^{d}, et éventuellement un groupe choisi parmi un halogène, -OR^{d}, hydrazine, -CF₃ et-CN,
- R⁵ et R⁶, identiques ou différents représentent un hydrogène, un halogène, un groupe en C1 à C15 comprenant une fonction aldéhyde, cétone, acide carboxylique ou ester, un nitrile, -SO₃Na, un groupe vinyle éventuellement substitué par un groupement cétone, ester ou aromatique, une imine substituée par un groupement alkyle ou aromatique, un groupe alcyne éventuellement substitué par un groupement alkyle ou un aromatique, SPh, un chalcogène aromatique (SePh, TePh), un amide, un groupe aryle ou hétéroaryle en C5 à C7, éventuellement substitué par au moins un groupe choisi parmi un halogène, -NR^{c}R^{d}, -OR^{d}, hydrazine, -CF₃ et-CN,
- optionnellement R³ et R⁵ et/ou R⁴ et R⁶ sont liés de manière covalente et forment ensemble un groupe aryle ou hétéroaryle en C5 à C7, éventuellement substitué par au moins un groupe choisi parmi un halogène, -NR^{c}R^{d}, -OR^{d}, hydrazine, -CF₃ et -CN,
- R^{c} et R^{d}, identiques ou différents, représentent un hydrogène ou une chaine alkyle, linéaire ou ramifiée en C1 à C3,
- R^{a} et R^{b}, identiques ou différents, représentent :
• un halogène, de préférence choisi dans le groupe comprenant le fluor et le chlore,
• un groupe en C1 à C50, aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O ,N, P et/ou S, de préférence sous la forme d'une ou plusieurs fonctions hydrophiles choisies parmi les fonctions ammonium quaternaire, sulfate, sulfonate et phosphonate,
• un groupe en C1 à C50, aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O, N, P et/ou S, de préférence sous la forme d'une ou plusieurs fonctions bioconjugables choisies parmi amine, acide carboxylique, ester activé de type N-hydrosuccinimide, pentafluorophényle, tetrafluorophenyle, squarate et plus particulièrement diéthylsquarate, maléimide, thiol, isothiocyanate, isocyanate, oxadiazolyl méthyl sulfone, azoture, tetrazine substituée ou non substituée, triazole, trans-cyclooctène, cyclooctyne et plus particulièrement dibenzocyclooctyne et bicyclononyne et un complexe PPh₂AuCl,
• un vecteur biologique couplé de manière covalente via un groupe en C1 à C50, aliphatique ou hétéroaliphatique, linéaire ou ramifié, saturé ou insaturé, comprenant éventuellement un ou plusieurs groupes aromatiques ou hétéroaromatiques, comprenant éventuellement un ou plusieurs hétéroatomes choisis parmi O, N, P et/ou S, de préférence sous la forme d'une ou plusieurs fonctions bioconjugables,
• un complexe métallique à visée thérapeutique, formé d'un agent chélatant et d'un métal,
• un complexe radiométallique, formé d'un agent chélatant et d'un radiométal, ou
• une molécule dont le diamètre hydrodynamique est inférieur à 10 nm, un peptide cyclique ou linéaire, un anticorps, un fragment d'anticorps, un nanobody, un affibody, un aptamère, une courte séquence d'ADN ou d'ARN, un sucre, un polysaccharide, un acide aminé, une vitamine, une molécule de type AMD3100, un ligand de la PSMA, un stéroïde, un acide gras, une polyamine, un polyphénol, une base de l'ADN ou un dérivé de la caféine.

2. Utilisation selon la revendication 1, dans laquelle le composé fluorophore est choisi parmi les composés de formule I dans laquelle R⁵ et R⁶ sont un hydrogène.

3. Utilisation selon l'une quelconque des revendications précédentes, dans laquelle le composé fluorophore est choisi parmi les composés de formule I dans laquelle R¹ et R², identiques ou différents, représentent un groupe aryl en C5 à C7 substitué par un groupe -NR^{c}R^{d} et éventuellement un groupe choisi parmi un halogène, -OR^{d}, hydrazine, -CF₃ et -CN.

4. Utilisation selon l'une quelconque des revendications précédentes, dans laquelle le composé fluorophore est choisi parmi les composés de formule I dans laquelle R¹ et/ou R² représentent un groupe phényle substitué par un groupe -NR^{c}R^{d}, de préférence en position para.

5. Utilisation selon l'une quelconque des revendications précédentes, dans laquelle le composé fluorophore est choisi parmi les composés de formule I dans laquelle R^{a} et R^{b}, identiques ou différents, sont choisis parmi un halogène, de préférence un fluor, parmi les groupes hydrophiles de formules suivantes : 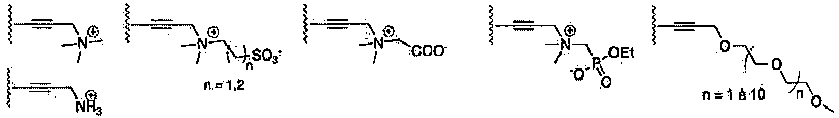 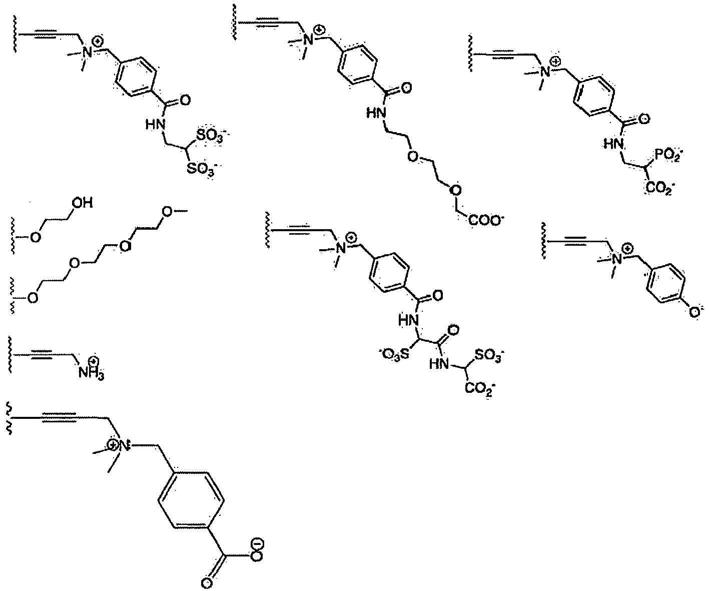 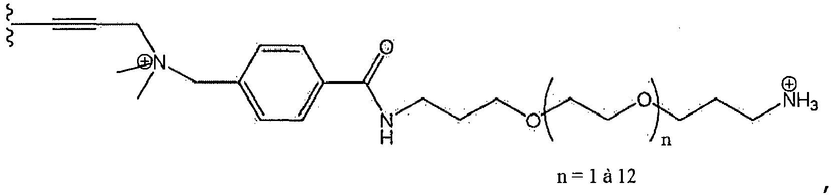 parmi les groupes comprenant une fonction bioconjugable de formules suivantes : 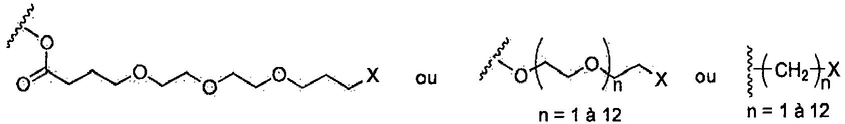 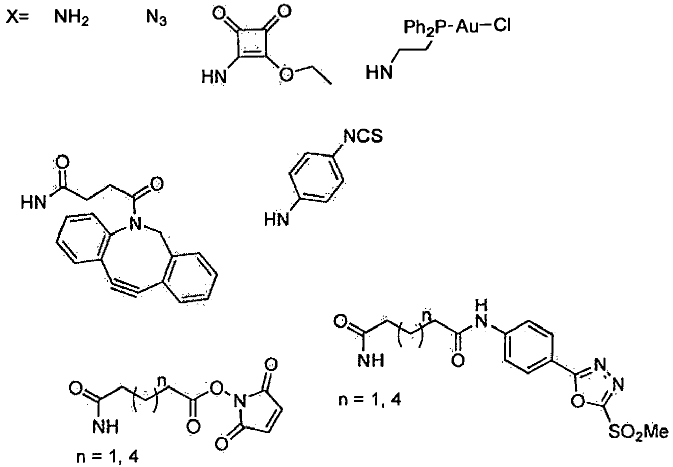 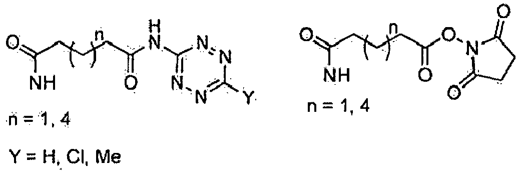 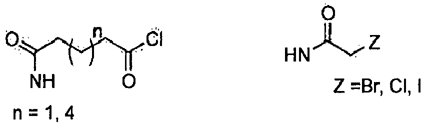 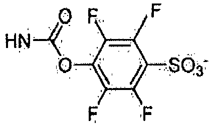 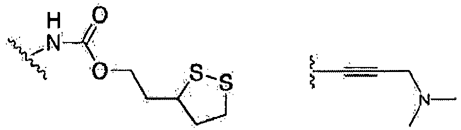 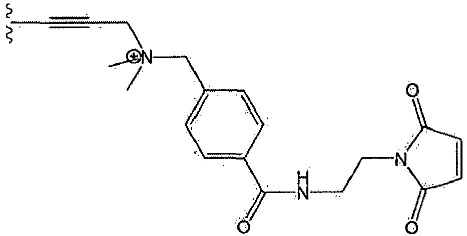 -NH₂ et -Si(OMe)₃, PPh₂-Au(I), PPh₂-Pt(II), PPh₂-Pt(IV), phneylpyridine-Au(III), DOTA-In(III), DOTAGA-In(III), NODAGA-Cu(II), NODAGA-Ga(III), ou parmi c(RGDfK) ciblant l'intégrine αᵥβ₃, ATWLPPR ciblant la neuropiline, anti-CD44, un thioglucose, un thioglucose peracétylé, l'acide folique, un ligand de type AMD3100, un ligand de la PSMA, la spermine, la spermidine, la cadavérine, la putrécine, le resvératrol, une base de l'ADN, la caféine et la progestérone.

6. Utilisation selon l'une quelconque des revendications précédentes, dans laquelle le composé fluorophore est choisi parmi les composés fluorophores de formule II : 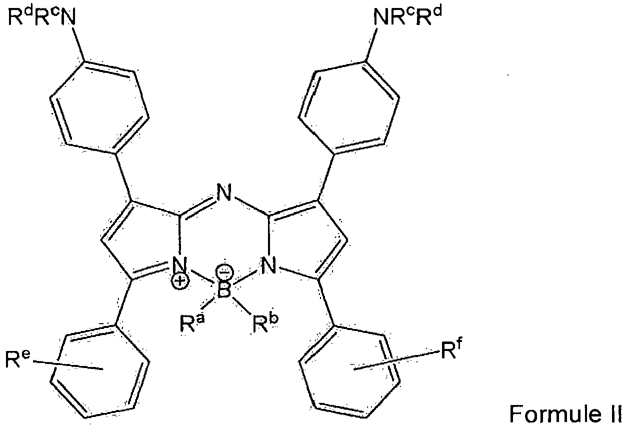 dans laquelle,
- Ra, Rb, Rc, Rd, ont les définitions données ci-dessus,
- Re et Rf, identiques ou différents, représente au moins un groupe parmi un hydrogène, un halogène, -NR^{c}R^{d}, -OR^{d}, hydrazine, -CF₃ et -CN.

7. Utilisation selon l'une quelconque des revendications précédentes, dans laquelle le composé fluorophore est choisi parmi les composés de formule I ou II dans laquelle Rc et Rd sont -CH₃.

8. Utilisation selon l'une quelconque des revendications précédentes, dans laquelle le composé fluorophore est compris dans une composition comprenant en outre un excipient pharmaceutiquement acceptable et/ou un solvant.

9. Utilisation selon la revendication précédente, dans laquelle la composition a un pH compris dans un intervalle allant de 4 à 10, de préférence 6 à 8 ou encore à 7,4.

10. Utilisation selon l'une quelconque des revendications précédentes, dans laquelle le composé fluorophore est encapsulé.

11. Kit comprenant un système d'injection et une composition comprenant un composé fluorophore de formule I ou II tel que défini ci-dessus et un excipient pharmaceutiquement acceptable et/ou un solvant.

12. Méthode d'identification in vitro d'une cible biologique comprenant au moins les étapes de :
- marquage des cellules d'un échantillon prélevé ou cultivé avec une composition comprenant un composé fluorophore de formule I ou II tel que défini ci-dessus,
- mesure de la fluorescence dans la fenêtre optique comprise dans un intervalle allant de 1000 à 1700 nm, de préférence par microscopie, cytométrie de flux, imagerie optique par réflexion de fluorescence (2D ou 3D), sonde optique, et
- identification des cellules ciblées.
